# EUROPEAN PATENT SPECIFICATION

(11) **EP 3 815 728 B1**
(45) Date of publication and mention of the grant of the patent: **19.10.2022**
(21) Application number: 20192654.0
(22) Date of filing: 25.08.2020
(51) Int. Cl.: A61M 13/00, B01D 46/00

(54) **INTEGRATED INTRACAVITY GAS CIRCULATION TREATMENT DEVICE FOR LAPAROSCOPIC SURGERY**
BEHANDLUNGSVORRICHTUNG ZUR INTEGRIERTEN INTRAKAVITALEN GASZIRKULATION FÜR LAPAROSKOPISCHE CHIRURGIE
DISPOSITIF DE TRAITEMENT DE LA CIRCULATION GAZEUSE INTRACAVITAIRE INTÉGRÉ POUR LA CHIRURGIE LAPAROSCOPIQUE

(30) Priority: 29.10.2019 CN 201911034517
(43) Date of publication of application: 05.05.2021
(73) Proprietor: Simai Co., Ltd., 519000 Zhuhai Guangdong (CN)
(72) Inventor: LIN, Min, Zhuhai, Guangdong 519000 (CN); ZHUO, Wenshuai, Zhuhai, Guangdong 519000 (CN)
(74) Representative: Zaboliene, Reda

(56) References cited:
- WO-A1-2018/039239
- WO-A1-2018/081587
- CN-U- 208 301 736

## Description

### Technical Field

The disclosure relates to the technical field of medical apparatuses and instruments, and in particular to an integrated intracavity gas circulation treatment device for a laparoscopic surgery.

### Background

Along with the advancement of the laparoscopic surgery technology, the laparoscopic minimally invasive surgery technology is promoted. When devices, such as an electrotome, are used for incision or electrocoagulation during a surgery process, a larger amount of smoke may be generated, this smoke may blur a mirror surface of an endoscope, and then it is caused that display content transmitted to a displayer through a camera system becomes blurred, it is the most annoying problem of a laparoscopic surgery. Therefore, this smoke must be removed in time.

An existing common smoke removing method is that the smoke is extracted by using a negative pressure device of a hospital. Most of the extracted smoke is CO₂ and odorous smoke gas, which is directly discharged after extraction. While air pollution is caused, an air pressure in an abdominal cavity is also reduced, it may not meet requirements of an abdominal space in a surgery, and a smoothness degree of the surgery may be directly affected. Therefore, a patent document CN201720129500.4 discloses a reflux aspirator, reference to Fig. 1 for details. The reflux aspirator includes a first filtering bottle 1 (liquid storage bottle), a second filtering bottle 2 (safety bottle), a filter 3 and a piston pump 4, herein, a connecting pipe 5 is connected between the first filtering bottle 1 and the second filtering bottle 2, a connecting pipe 6 is connected between the second filtering bottle 2 and the filter 3, and a connecting pipe 7 is connected between the filter 3 and the piston pump 4.

It may be observed from the above that the reflux aspirator includes three filtering parts (the first filtering bottle 1, the second filtering bottle 2 and the filter 3) and a driving part (the piston pump 4), liquid and gas in the abdominal cavity are extracted by the piston pump 4, and filtered through the first filtering bottle 1, the second filtering bottle 2 and the filter 3 successively. In use, each of the above components is independently installed mutually, but communicated through each connecting pipe (5/6/7), therefore the following disadvantages may be existent: 1. an overall structure of the reflux aspirator is quite complicated and cumbersome, a volume is larger, and it is very inconvenient for use and operation by a medical worker during the laparoscopic surgery; 2. multiple filtering parts and driving parts are connected by the connecting pipes (5/6/7), and a disadvantage of poor connection airtightness is existent; and 3. the multiple filtering parts and driving parts are connected by the connecting pipes (5/6/7) and repeatedly used, it may also cause a problem that a filtered gas is cross-infected again by bacteria, and a risk of the surgery is directly increased.

WO 2018/081587 A1 disclosed an insufflation gas filtration apparatus for use in laparoscopic surgery. The insufflation gas filtration apparatus includes a filter cartridge having an inlet, an outlet, a filter medium and an impeller. The filter cartridge is releasably coupleable to a reusable motor base. An insufflation gas filtration system and a method of use of the insufflation gas filtration apparatus are also disclosed. Specifically, the system employed in providing and maintaining pneumoperitoneum within a body cavity C of a patient includes two-part filtration apparatus 12, comprising filter cartridge 14 and motor base 16. The filtration apparatus 12 is provided with the internal working mechanism of disposable filter cartridge 14 and reusable brushless motor base 16. Within reusable filter cartridge 14, filter medium 74 is separated from centrifugal impeller 76 by septum 78. Shell 84 of filter cartridge 14 includes inlet 102, to which fitting 42 is connected, and outlet 104, to which fitting 18 is connected.

### Summary

A purpose of the disclosure is to provide an integrated intracavity gas circulation treatment device for a laparoscopic surgery, as to solve the above problems in an existing technology. The integrated intracavity gas circulation treatment device is capable of realizing integrated installation of a filtering unit and a driving pump unit, skillful in design, compact in structure, and small in occupied space. In a process of the laparoscopic surgery, the integrated intracavity gas circulation treatment device is simply and conveniently used and operated by a medical worker. In the whole surgery process, stability of an air pressure in the abdominal cavity is high, an effect of gas circulation filtering is good, surgery efficiency and quality are improved, and it is guaranteed that a contaminated and non-sterilizable work piece (such as the driving pump) may not be repeatedly used, so real zero infection is achieved.

In order to achieve the above purpose, the disclosure provides the following technical schemes.

An integrated intracavity gas circulation treatment device for a laparoscopic surgery, including a filtering unit and a driving pump unit, herein, the filtering unit is provided with an air inlet channel, the air inlet channel may be communicated with a human abdominal cavity; the driving pump unit is installed in the filtering unit, and communicated with the filtering unit; and the driving pump unit is provided with an air exhausting channel, the air exhausting channel may be communicated with the human abdominal cavity.

Further, the filtering unit includes a filtering cavity and a filter element, the filter element is circumferentially installed on a cavity wall of the filtering cavity, the air inlet channel is installed outside the filtering cavity, and the air inlet channel is capable of enabling the filtering cavity to be communicated with the human abdominal cavity; and the driving pump unit includes a sealing cavity and a driving mechanism, the driving mechanism is installed in the sealing cavity, the sealing cavity is positioned in the filtering cavity, and the filtering cavity is communicated with the sealing cavity, a cavity wall of the sealing cavity is provided with the air exhausting channel, the air exhausting channel is capable of enabling the sealing cavity to be communicated with the human abdominal cavity.

Further, the cavity wall of the filtering cavity is provided with a communicating port, the filter element is circumferentially installed on the cavity wall of the filtering cavity, the air inlet channel is capable of enabling the communicating port to be communicated with the human abdominal cavity.

Further, the sealing cavity includes a first cavity cover body and a second cavity cover body, an upper opening of the first cavity cover body is in sealed-connection with a lower opening of the second cavity cover body, and an upper part of the second cavity cover body is provided with the air exhausting channel protruded outwards.

Further, a cavity wall of the first cavity cover body is provided with a channel port, and/or a cavity wall of the second cavity cover body is provided with the channel port, the channel port is communicated with the communicating port.

Further, the filtering unit includes a third cavity cover body, a fourth cavity cover body, an inner holder, an outer holder and a sealing cover, the filter element is sealed and installed between the inner holder and the outer holder, one end of each of the inner holder and the outer holder is in sealed-connection with a cover bottom of the third cavity cover body, and the other end of each of the inner holder and the outer holder is in sealed-connection with the sealing cover, the cover bottom of the third cavity cover body is in sealed-connection with the cover bottom of the first cavity cover body, and an upper opening of the third cavity cover body is in sealed-connection with a lower opening of the fourth cavity cover body.

Further, the sealing cover is provided with an opening communicated with the air exhausting channel, and an inner wall of the opening is in sealed-connection with an outer wall of the air exhausting channel.

Further, the fourth cavity cover body is provided with a first separating part protruded inwards, the fourth cavity cover body is provided with an air exhausting port and an air inlet port protruded outwards; the sealing cover is provided with a second separating matched and connected with the first separating part, and a cavity formed after the third cavity cover body is in sealed-connection with the fourth cavity cover body is divided into an air exhausting cavity and the air inlet channel by two parties; the air exhausting port, the air exhausting cavity and the air exhausting channel are successively communicated; and the air inlet port is communicated with the air inlet channel.

Further, the driving pump unit further includes a driving connecting part, the driving connecting part penetrates into the sealing cavity and is in sealed-connection with a cavity wall of the sealing cavity, an inner end part of the driving connecting part is connected to the driving mechanism, and an outer end part of the driving connecting part is detachably connected to an external driving device.

Further, the driving connecting part is in sealed-connection with the cavity wall of the sealing cavity through magnetic fluid.

Beneficial effects of the disclosure are as follows.
1. Based on the integrated intracavity gas circulation treatment device provided by the disclosure, the driving pump unit is installed in the filtering unit, and communicated with the filtering unit mutually, the integrated installation of the filtering unit and the driving pump unit is realized, the structure is compact, and the volume is small. The medical worker only needs to enable the filtering unit and the driving pump unit to be respectively communicated with the human abdominal cavity (for example, respectively communicated with puncture outfits used in different surgeries), the use and operation are quite simple and convenient. When the integrated intracavity gas circulation treatment device is used, smoke generated in the human abdominal cavity during the surgery is extracted under the effect of a driving force (for example, a suction force) of the driving pump unit, and filtered (for example, filtration sterilization) by the filtering unit, the filtered gas is injected into the human abdominal cavity again through the driving pump unit, so that gas circulation in the human abdominal cavity is realized, and the gas circulation in the abdominal cavity is high in stability, good in effect, and a success rate of the surgery is improved.
2. Based on the integrated intracavity gas circulation treatment device provided by the disclosure, the integrated intracavity gas circulation treatment device is simply and conveniently used and operated by the medical worker. In the whole surgery process, stability of an air pressure in the abdominal cavity is high, an effect of gas circulation filtering is good, surgery efficiency and quality are improved, and it is guaranteed that a contaminated and non-sterilizable work piece (such as the driving pump) may not be repeatedly used, so real zero infection is achieved.

### Brief Description of the Drawings

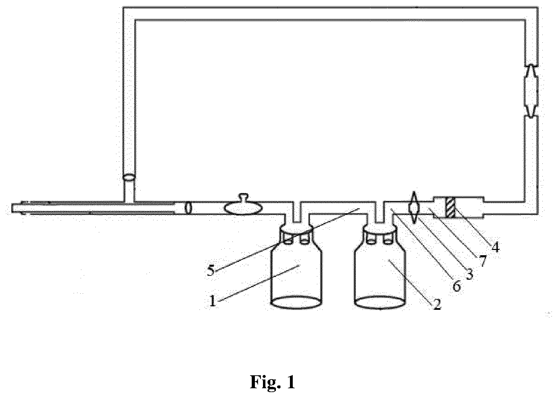

Fig. 1 is a structure schematic diagram of a reflux aspirator in an existing technology;
Fig. 2 is a circulation theory schematic diagram of the integrated intracavity gas circulation treatment device;
Fig. 3 is an overall structure schematic diagram of the integrated intracavity gas circulation treatment device;
Fig. 4 is an overall structure schematic diagram of a driving pump unit;
Fig. 5 is an internal structure diagram of a first cavity cover body;
Fig. 6 is an overall structure schematic diagram of a filtering unit;
Fig. 7 is a structure decomposition schematic diagram of the filtering unit; and
Fig. 8 is a section diagram of the integrated intracavity gas circulation treatment device.

In the drawings, 100-Filtering unit, 10-Filtering cavity, 11-Third cavity cover body, 111-Cover bottom, 12-Fourth cavity cover body, 121-First separating part, 122-Air exhausting port, 123-Air inlet port, 13-Inner holder, 131-Communicating port, 14-Outer holder, 141-Communicating port, 15-Sealing cover, 151-Opening, 152-Second separating part, 16-Air exhausting cavity, 17-Sealing ring, 20-Filter element, 30-Air inlet channel, 200-Driving pump unit, 40-Sealing cavity, 41-First cavity cover body, 410-Upper opening, 411-Channel port, 412-Cover bottom flange, 42-Second cavity cover body, 420-Lower opening, 50-Driving mechanism, 60-Air exhausting channel, 70-Driving connecting part, 80-Bolt, 1-First filtering bottle, 2-Second filtering bottle, 3-Filter, 4-Piston pump, 5-Connecting pipe, 6-Connecting pipe, 7-Connecting pipe, and F-Human abdominal cavity.

### Detailed Description of the Embodiments

Technical schemes in embodiments of the disclosure are clearly and completely described below in combination with drawings in the embodiments of the disclosure. Apparently, the embodiments described are only a part of the embodiments of the disclosure, rather than all of the embodiments. Based on the embodiments of the disclosure, all other embodiments obtained by those of ordinary skill in the art without creative work shall fall within the scope of protection of the disclosure.

The present embodiment provides an integrated intracavity gas circulation treatment device for a laparoscopic surgery. The integrated intracavity gas circulation treatment device is generally cylindrical, or may be cube-shaped, and is not specifically limited. It is used for an abdominal surgery in the medical field, and specifically refers that smoke in a human abdominal cavity F is extracted, and the gas is injected into the human abdominal cavity F again after being filtered.

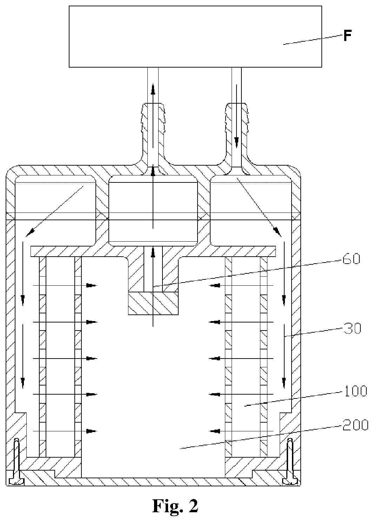

Reference to Fig. 2 for details, in the present embodiment, the integrated intracavity gas circulation treatment device includes a filtering unit 100 and a driving pump unit 200, the driving pump unit 200 is installed in the filtering unit 100, and the driving pump unit 200 is communicated with the filtering unit 100 mutually, the integrated installation of the filtering unit 100 and the driving pump unit 200 is realized, the structure is compact, and the volume is small. The medical worker only needs to enable the filtering unit 100 and the driving pump unit 200 to be respectively communicated with the human abdominal cavity F, specifically, the filtering unit 100 is connected with a tool (for example, a surgical instrument such as a puncture outfit) commonly used in a surgery through an air inlet channel 30, as to communicate into the human abdominal cavity F, the driving pump unit 200 is connected with a tool (for example, another puncture outfit) commonly used in the surgery through an air exhausting channel 60, as to communicate into the human abdominal cavity F, the use and operation are quite simple and convenient. The gas circulation in the abdominal cavity is high in stability, good in effect, and a success rate of the surgery is improved.

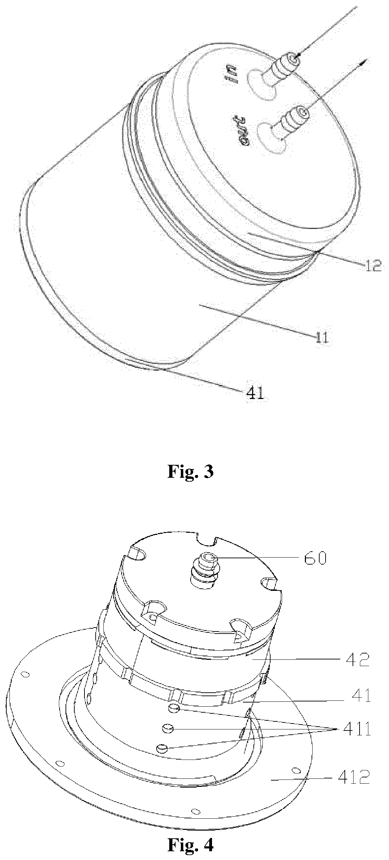

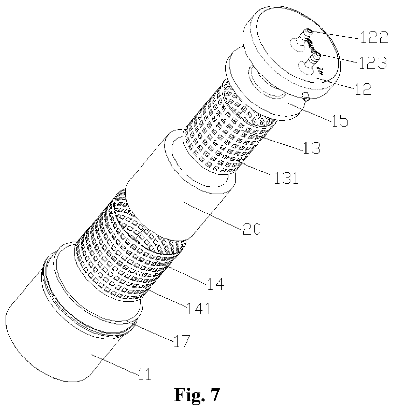

Specifically, reference to Fig. 3 to Fig. 8 for details, the filtering unit 100 includes a filtering cavity 10 and a filter element 20, herein, a cavity wall of the filtering cavity 10 is circumferentially provided with a communicating port 131 (141), the filter element 20 is circumferentially installed on the above cavity wall, the air inlet channel 30 is installed outside the filtering cavity 10, the air inlet channel 30 is capable of enabling the communicating port 131 (141) to be communicated with the human abdominal cavity F, as to enable the filtering cavity 10 to be communicated with the human abdominal cavity F; and the driving pump unit 200 includes a sealing cavity 40 and a driving mechanism 50, herein, the driving mechanism 50 is accommodated in the sealing cavity 40, the sealing cavity 40 is accommodated in the filtering cavity 10, and the filtering cavity 10 and the sealing cavity 40 are communicated mutually, specifically, a cavity wall of the sealing cavity 40 is provided with a channel port 411, the cavity wall of the sealing cavity 40 is provided with the air exhausting channel 60, and the air exhausting channel 60 is capable of enabling the sealing cavity 40 to be communicated with the human abdominal cavity F.

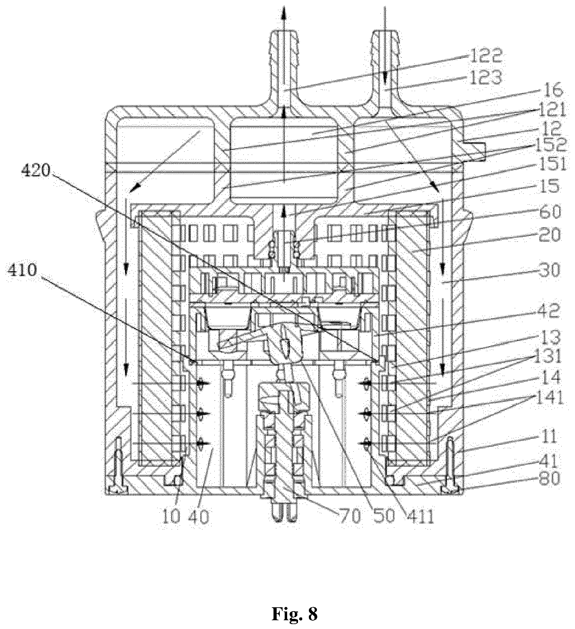

Based on the integrated intracavity gas circulation treatment device, smoke generated in the human abdominal cavity F during the surgery is extracted under the effect of a driving force (for example, a suction force) of the driving pump unit 200, and filtered (for example, filtration sterilization) by the filtering unit 100, the filtered gas is injected into the human abdominal cavity F again through the driving pump unit 200, so that gas circulation (reference to an arrow in Fig. 8 for details) in the human abdominal cavity F is realized, it specifically refers that the driving mechanism 50 (may be a mechanism such as a piston pump) in the sealing cavity 40 may produce the driving force (for example, the suction force), the filtering cavity 10 and the sealing cavity 40 are communicated mutually, at the same time, the filtering cavity 10 is connected with the tool (for example, the surgical instrument such as the puncture outfit) commonly used in the surgery through the air inlet channel 30, as to communicate into the human abdominal cavity F, therefore the smoke generated in the human abdominal cavity F during the surgery may be extracted under the effect of the suction force, and filtered (for example, the filtration sterilization) by the filter element 20 circumferentially installed on the filtering cavity 10, at the same time, the sealing cavity 40 is connected with the tool (for example, the surgical instrument such as the puncture outfit) commonly used in the surgery through the air exhausting channel 60, as to communicate into the human abdominal cavity F, therefore the filtered gas is injected into the human abdominal cavity F again through the sealing cavity 40, so that the gas circulation in the human abdominal cavity F is realized.

Specifically, in the present embodiment, the driving pump unit 200 includes the sealing cavity 40, the driving mechanism 50 and a driving connecting part 70, the driving mechanism 50 is accommodated in the sealing cavity 40, the cavity wall of the sealing cavity 40 is provided with the air exhausting channel 60, and the cavity wall thereof is provided with the channel port 411, the filtering cavity 10 and the sealing cavity 40 are communicated through the channel port 411, the air exhausting channel 60 is communicated with an inner cavity of the sealing cavity 40 and the surgical instrument, as to communicate into the human abdominal cavity F.

Specifically, the driving mechanism 50 is accommodated in the sealing cavity 40, and may be communicated with the human abdominal cavity F and the filtering cavity 10 and connected with an external driving device (may be a driving motor), so that the smoke in the human abdominal cavity F is extracted into the filtering cavity 10 by the produced suction force, the gas filtered by the filtering cavity 10 is extracted into the sealing cavity 40, and injected into the human abdominal cavity F again.

In order to conveniently install the driving mechanism 50 in the sealing cavity 40, the sealing cavity 40 includes a first cavity cover body 41 and a second cavity cover body 42, the second cavity cover body 42 further includes a cover bottom flange 412, a cavity wall of the first cavity cover body 41 is provided with the channel port 411, as to communicate with the filtering cavity 10, the air exhausting channel 60 is outwards protruded and installed on the top of the second cavity cover body 42 so as to communicate into the human abdominal cavity F, while the driving mechanism 50 is installed in position, the upper opening 410 of the first cavity cover body 41 is in sealed-connection with the lower opening 420 of the second cavity cover body 42, it may be sealed and connected by a high-temperature-resistant sealant, or may be sealed by using a fixing mode of a sealing ring and a screw, specifically, an inner cavity of the first cavity cover body 41 is provided with a screw hole, correspondingly, the sealing ring and an inner cavity of the second cavity cover body 42 are provided with the screw holes too, and the first cavity cover body 41 is in sealed-connection with the second cavity cover body 42 by using a long screw.

In addition, the driving mechanism 50 is installed in the sealing cavity 40, and detachably connected with the external driving device by the driving connecting part 70 so as to produce the suction force, the above specific structure and principle are in accordance with a patent CN201821266360.6 (detachable sealing air pump). Specifically, the integrated intracavity gas circulation treatment device may be connected to the external driving device and detached from the external driving device at any time, and it is more convenient for replacement of the integrated intracavity gas circulation treatment device, requirements of one-time use are achieved, because the device may not be circularly used after being used for some patients with infectious diseases, a phenomenon of bacteria cross-infection is prevented.

Herein, the filtering unit 100 in the present embodiment includes the filtering cavity 10 and the filter element 20, the filter element 20 is circumferentially installed on the cavity wall of the filtering cavity 10, the air inlet channel 30 is installed outside the filtering cavity 10, the cavity wall of the filtering cavity 10 is provided with the communicating port 131 (141), and the communicating port 131 (141) is communicated with the channel port 411, preferably, the communicating port 131 (141) is installed corresponding to the channel port 411, the air inlet channel 30 is communicated with the inner cavity of the filtering cavity 10 and the surgical instrument, as to communicate into the human abdominal cavity F, the communicating port 131 (141) is used to communicate with the sealing cavity 40 of the driving pump unit 200.

Specifically, in order to improve a filtering effect, the filter element 20 may include a multi-layer filter element of which a filter capacity is progressed layer by layer from outside to inside, namely the smoke extracted from the human abdominal cavity F may be successively filtered by the above multi-layer filter element layer by layer, specifically, impurities, such as water, oil, blood and smashed particles, are firstly filtered, and then harmful substances and the smoke generated in work of devices such as an electrotome are filtered, for example, a sulfide, a chloride, bacteria, microorganism particles, and the smoke.

In addition, preferably, the whole filter element 20 is tube-shaped, the multi-layer filter elements are sealed and connected by a high-temperature-resistant sealant, specifically, an edge area of an outer filtering surface of the first layer of the filter element rolled into a tube shape is coated with the high-temperature-resistant sealant, and then the second layer of the filter element is attached, and so on, a situation that skipping filtering happens to a filtering process is prevented, herein the above high-temperature-resistant sealant is a commonly-used two-component adhesive, for example, a two-component epoxy adhesive, and a two-component polyurethane adhesive, as to ensure that it is non-toxic and harmless to a human body, high-temperature-resistant, and may achieve an effect of efficient sealing.

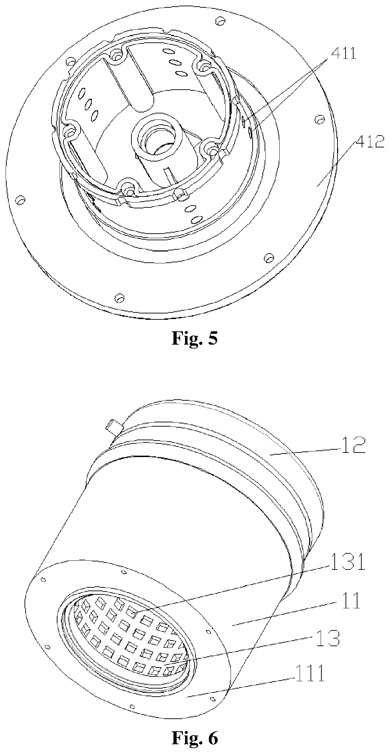

Reference to Fig. 6 to Fig. 8 for details, in order to conveniently install the filter element 20, the filtering unit 100 includes a third cavity cover body 11, a fourth cavity cover body 12, an inner holder 13, an outer holder 14 and a sealing cover 15, herein, one end of each of the inner holder 13 and the outer holder 14 is in sealed-connection with a cover bottom 111 of the third cavity cover body 11 by welding, and the other end of each of the inner holder 13 and the outer holder 14 is in sealed-connection with the sealing cover 15 by welding, it may be sealed and connected by the high-temperature-resistant sealant, a wall of the inner holder 13 is provided with the communicating port 131, and a wall of the outer holder 14 is provided with the communicating port 141, the cover bottom 111 of the third cavity cover body 11 is in sealed-connection with the cover bottom 111 of the first cavity cover body 41 through a sealing ring 17 and a screw 80, as to form the filtering cavity 10, the inner holder 13 and the outer holder 14 form the cavity wall of the filtering cavity 10, and the filter element 20 is sealed and installed between the inner holder 13 and the outer holder 14.

Continuously reference to Fig. 6 to Fig. 8, an upper opening of the third cavity cover body 11 is in sealed-connection with a lower opening of the fourth cavity cover body 12 by welding, it may be sealed and connected by the high-temperature-resistant sealant, the fourth cavity cover body 12 is provided with a first separating part 121 protruded outwards, the first separating part 121 is ring-protruded, the fourth cavity cover body 12 is provided with an air exhausting port 122 and an air inlet port 123 protruded outwards, the air exhausting port 122 and the air inlet port 123 are interval-installed, correspondingly, the sealing cover 15 is provided with a second separating part 152 matched and connected with the first separating part 121, the second separating part 152 is ring-protruded too, the upper opening of the third cavity cover body 11 is in sealed-connection with the lower opening of the fourth cavity cover body 12 by welding, at the same time, the first separating part 121 is also in sealed-connection with the second separating part 152 by welding, so that an inner cavity formed after the third cavity cover body 11 is in sealed-connection with the fourth cavity cover body 12 is divided into an air exhausting cavity 16 and the air inlet channel 30, the air inlet port 123 is communicated with the air inlet channel 30, the air inlet channel 30 is communicated with the communicating port 131(141), and communicated with the channel port 411, so that the smoke generated in the human abdominal cavity F during the surgery is extracted by the driving pump unit 200, the air exhausting port 122, the air exhausting cavity 16 and the air exhausting channel 60 are successively communicated, so that the filtered gas is injected into the human abdominal cavity F again by the driving pump unit 200.

The sealing cover 15 is provided with an opening 151 communicated with the air exhausting channel 60, an outer wall of the air exhausting channel 60 penetrates into the opening 151, and the outer wall of the air exhausting channel 60 is in sealed-connection with an inner wall of the opening 151 by the sealing ring, so that it is guaranteed that the filtered gas is smoothly and stably injected into the human abdominal cavity F again by the driving pump unit 200.

The above embodiments are only used to illustrate the technical schemes of the disclosure and not to limit. Although the disclosure is described in detail with reference to the preferable embodiments, it should be understood by those skilled in the art that modifications or equivalent replacements may be made to the technical schemes of the disclosure. The invention is defined by the appended claims.

## Claims

1. An integrated intracavity gas circulation treatment device for a laparoscopic surgery, the integrated intracavity gas circulation treatment device for the laparoscopic surgery comprises a filtering unit (100) and a driving pump unit (200), wherein :
the filtering unit (100) is provided with an air inlet channel (30), the air inlet channel (30) can be communicated with a human abdominal cavity (F);
the driving pump unit (200) is installed in the filtering unit (100), and communicated with the filtering unit (100); and
the driving pump unit (200) is provided with an air exhausting channel (60), the air exhausting channel (60) can be communicated with the human abdominal cavity (F) the filtering unit (100) comprises a filtering cavity (10) and a filter element (20), the filter element (20) is circumferentially installed on a cavity wall of the filtering cavity (10), the air inlet channel (30) is installed outside the filtering cavity (10), and the air inlet channel (30) is capable of enabling the filtering cavity (10) to be communicated with the human abdominal cavity (F); and
the driving pump unit (200) comprises a sealing cavity (40) and a driving mechanism (50), the driving mechanism (50) is installed in the sealing cavity (40), the sealing cavity (40) is positioned in the filtering cavity (10), and the filtering cavity (10) is communicated with the sealing cavity (40), a cavity wall of the sealing cavity (40) is provided with the air exhausting channel (60), the air exhausting channel (60) is capable of enabling the sealing cavity (40) to be communicated with the human abdominal cavity (F);
the cavity wall of the filtering cavity (10) is provided with a communicating port (131), the air inlet channel (30) is capable of enabling the communicating port (131) to be communicated with the human abdominal cavity (F);
the sealing cavity (40) comprises a first cavity cover body (41) and a second cavity cover body (42), which together form a cylindrical cavity, wherein the first cavity cover body (41) and the second cavity cover body (42) have a hollow cylindrical shape at least in sections, the hollow cylindrical sections being connected to each other, and the first cavity cover body (41) is arranged underneath the second cavity cover body (42), an upper opening (410) of the first cavity cover body (41) is in sealed-connection with a lower opening (420) of the second cavity cover body (42), and an upper part of the second cavity cover body (42) is provided with the air exhausting channel (60) protruded outwards, wherein the air exhausting channel (60) is installed on the top of the second cavity cover body (42);
a cavity wall of the first cavity cover body (41) is provided with a channel port (411), and/or a cavity wall of the second cavity cover body (42) is provided with the channel port (411), the channel port (411) is communicated with the communicating port (131);
the filtering unit (100) comprises a third cavity cover body (11), a fourth cavity cover body (12), an inner holder (13), an outer holder (14) and a sealing cover (15), the filter element (20) is sealed and installed between the inner holder (13) and the outer holder (14), one end of each of the inner holder (13) and the outer holder (14) is in sealed-connection with a cover bottom (111) of the third cavity cover body (11), and the other end of each of the inner holder (13) and the outer holder (14) is in sealed-connection with the sealing cover (15), the cover bottom (111) of the third cavity cover body (11) is in sealed-connection with the cover bottom (111) of the first cavity cover body (41), and an upper opening of the third cavity cover body (11) is in sealed-connection with a lower opening of the fourth cavity cover body (12);
the sealing cover (15) is provided with an opening (151) communicated with the air exhausting channel (60), and an inner wall of the opening (151) is in sealed-connection with an outer wall of the air exhausting channel (60);
the fourth cavity cover body (12) is provided with a first separating part (121) protruded inwards, the fourth cavity cover body (12) is provided with an air exhausting port (122) and an air inlet port (123) protruded outwards; the sealing cover (15) is provided with a second separating part (152) matched and connected with the first separating part (121), and a cavity formed after the third cavity cover body (11) is in sealed-connection with the fourth cavity cover body (12) is divided into an air exhausting cavity (16) and the air inlet channel (30) by two parties; the air exhausting port (122), the air exhausting cavity (16) and the air exhausting channel (60) are successively communicated; and the air inlet port (123) is communicated with the air inlet channel (30).

2. The integrated intracavity gas circulation treatment device for the laparoscopic surgery as claimed in claim 1, wherein :
the driving pump unit (200) further comprises a driving connecting part (70), the driving connecting part (70) penetrates into the sealing cavity (40) and is in sealed-connection with a cavity wall of the sealing cavity (40), an inner end part of the driving connecting part (70) is connected to the driving mechanism (50), and an outer end part of the driving connecting part (70) is detachably connected to an external driving device.

3. The integrated intracavity gas circulation treatment device for the laparoscopic surgery as claimed in claim 2, wherein : the driving connecting part (70) is in sealed-connection with the cavity wall of the sealing cavity (40) through magnetic fluid.

## Patentansprüche

1. Integrierte intrakavitäre Gaszirkulationsvorrichtung für die laparoskopische Chirurgie, wobei die integrierte intrakavitäre Gaszirkulationsvorrichtung für die laparoskopische Chirurgie eine Filtereinheit (100) und eine Antriebspumpeneinheit (200) umfasst, wobei
die Filtereinheit (100) mit einem Lufteinlasskanal (30) versehen ist, und der Lufteinlasskanal (30) mit der menschlichen Bauchhöhle (F) in Verbindung stehen kann;
die Antriebspumpeneinheit (200) in der Filtereinheit (100) installiert ist und mit der Filtereinheit (100) in Verbindung steht; und die Antriebspumpeneinheit (200) mit einem Luftauslasskanal (60) versehen ist, der Luftauslasskanal (60) mit der menschlichen Bauchhöhle (F) in Verbindung stehen kann, die Filtereinheit (100) einen Filterhohlraum (10) und ein Filterelement (20) umfasst, wobei das Filterelement (20) umlaufend an der Hohlraumwand des Filterhohlraums (10) installiert ist, der Lufteinlasskanal (30) außerhalb des Filterhohlraums (10) installiert ist, und der Lufteinlasskanal (30) in der Lage ist, eine Verbindung des Filterhohlraums (10) mit der menschlichen Bauchhöhle (F) zu ermöglichen;
und die Antriebspumpeneinheit (200) einen abgedichteten Hohlraum (40) und einen Antriebsmechanismus (50) umfasst, wobei der Antriebsmechanismus (50) im abgedichteten Hohlraum (40) installiert ist, der abgedichtete Hohlraum (40) im Filterhohlraum (10) positioniert ist, und der Filterhohlraum (10) mit dem abgedichteten Hohlraum (40) in Verbindung steht, eine Hohlraumwand des abgedichteten Hohlraums (40) mit dem Luftauslasskanal (60) versehen ist, der Luftauslasskanal (60) in der Lage ist, eine Verbindung des abgedichteten Hohlraums (40) mit der menschlichen Bauchhöhle (F) zu ermöglichen;
die Hohlraumwand des Filterhohlraums (10) mit einem Verbindungsanschluss (131) versehen ist, der Lufteinlasskanal (30) in der Lage ist, eine Verbindung des Verbindungsanschlusses (131) mit der menschlichen Bauchhöhle (F) zu ermöglichen;
der abgedichtete Hohlraum (40) ein erstes Hohlraumabdichtungsgehäuse (41) und ein zweitens Hohlraumabdichtungsgehäuse (42) umfasst, die zusammen einen zylindrischen Hohlraum bilden, wobei das erste Hohlraumabdichtungsgehäuse (41) und das zweite Hohlraumabdichtungsgehäuse (42) zumindest abschnittsweise eine hohlzylindrische Form aufweisen, wobei die hohlzylindrischen Abschnitte miteinander verbunden sind und das erste Hohlraumabdichtungsgehäuse (41) unterhalb des zweiten Hohlraumabdichtungsgehäuses (42) angeordnet ist, die obere Öffnung (410) des ersten Hohlraumabdichtungsgehäuses (41) in abgedichteter Verbindung mit der unteren Öffnung (420) des zweiten Hohlraumabdichtungsgehäuses (42) steht und der obere Teil des zweiten Hohlraumabdichtungsgehäuses (42) mit dem Luftauslasskanal (60) versehen ist, der nach außen vorsteht, wobei der Luftauslasskanal (60) auf der Oberseite des zweiten Hohlraumabdichtungsgehäuses (42) installiert ist; eine Hohlraumwand des ersten Hohlraumabdichtungsgehäuses (41) mit einer Kanalöffnung (411) versehen ist und/oder eine Hohlraumwand des zweiten Hohlraumabdichtungsgehäuses (42) mit der Kanalöffnung (411) versehen ist, wobei die Kanalöffnung (411) mit dem Verbindungsanschluss (131) in Verbindung steht;
die Filtereinheit (100) ein drittes Hohlraumabdichtungsgehäuse (11), ein viertes Hohlraumabdichtungsgehäuse (12), einen inneren Halter (13), einen äußeren Halter (14) und einen Dichtungsdeckel (15) umfasst, wobei das Filterelement (20) abgedichtet und zwischen dem inneren Halter (13) und dem äußeren Halter (14) installiert ist, ein Ende sowohl des inneren Halters (13) als auch des äußeren Halters (14) in abgedichteter Verbindung mit dem Gehäuseboden (111) des dritten Hohlraumabdichtungsgehäuses (11) steht und das andere Ende sowohl des inneren Halters (13) als auch des äußeren Halters (14) in abgedichteter Verbindung mit dem Dichtungsdeckel (15) steht, der Gehäuseboden (111) des dritten Hohlraumabdichtungsgehäuses (11) in abgedichteter Verbindung mit dem Gehäuseboden (111) des ersten Hohlraumabdichtungsgehäuses (41) steht und die obere Öffnung des dritten Hohlraumabdichtungsgehäuses (11) in abgedichteter Verbindung mit der unteren Öffnung des vierten Hohlraumabdichtungsgehäuses (12) steht; der Dichtungsdeckel (15) mit einer Öffnung (151) versehen ist, die mit dem Luftauslasskanal (60) in Verbindung steht, und die Innenwand der Öffnung (151) in abgedichteter Verbindung mit der Außenwand des Luftauslasskanals (60) steht;
das vierte Hohlraumabdichtungsgehäuse (12) mit einem nach innen vorstehenden ersten Trennteil (121) versehen ist, das vierte Hohlraumabdichtungsgehäuse (12) mit einer Luftauslassöffnung (122) und einer nach außen vorstehenden Lufteinlassöffnung (123) versehen ist; der Dichtungsdeckel (15) mit einem zweiten Trennteil (152) versehen ist, das mit dem ersten Trennteil (121) zusammenpasst und verbunden ist, und ein Hohlraum, der gebildet wird, nachdem das dritte Hohlraumabdichtungsgehäuse (11), das in abgedichteter Verbindung mit dem vierten Hohlraumabdichtungsgehäuse (12) steht, durch zwei Teile in einen Luftauslasshohlraum (16) und den Lufteinlasskanal (30) unterteilt wird; die Luftauslassöffnung (122), der Luftauslasshohlraum (16) und der Luftauslasskanal (60) sequentiell nacheinander in Verbindung stehen; und die Lufteinlassöffnung (123) mit dem Lufteinlasskanal (30) in Verbindung steht.

2. Integrierte intrakavitäre Gaszirkulationsvorrichtung für die laparoskopische Chirurgie nach Anspruch 1, wobei die Antriebspumpeneinheit (200) ferner einen Antriebsverbindungsteil (70) umfasst, wobei der Antriebsverbindungsteil (70) in den abgedichteten Hohlraum (40) eindringt und in abgedichteter Verbindung mit der Hohlraumwand des abgedichteten Hohlraums (40) steht, das innere Endteil des Antriebsverbindungsteils (70) mit dem Antriebsmechanismus (50) verbunden ist und das äußere Endteil des Antriebsverbindungsteils (70) lösbar mit der externen Antriebsvorrichtung verbunden ist.

3. Integrierte intrakavitäre Gaszirkulationsvorrichtung für die laparoskopische Chirurgie nach Anspruch 2, wobei: das Antriebsverbindungsteil (70) durch ein Ferrofluid in abgedichteter Verbindung mit der Hohlraumwand des abgedichteten Hohlraums (40) steht.

## Revendications

1. Dispositif intégré de traitement par circulation de gaz intracavité pour une chirurgie laparoscopique, le dispositif de traitement de circulation de gaz intracavitaire intégré pour une chirurgie laparoscopique comprend une unité de filtrage (100) et une unité de pompe d'entraînement (200), dans lequel;
L'unité de filtrage (100) est pourvue d'un canal d'entrée d'air (30), le canal d'entrée d'air (30) pouvant être mis en communication avec une cavité abdominale humaine (F) ;
l'unité de pompe d'entraînement (200) est installée dans l'unité de filtrage (100) et communique avec l'unité de filtrage (100) ; et l'unité de pompe d'entraînement (200) est pourvue d'un canal d'évacuation d'air (60), le canal d'évacuation d'air (60) peut être mis en communication avec la cavité abdominale humaine (F) l'unité de filtrage (100) comprend une cavité filtrante (10) et un élément filtrant (20), l'élément filtrant (20) est installé circonférentiellement sur une paroi de cavité de la cavité filtrante (10), le canal d'entrée d'air (30) est installé à l'extérieur de la cavité filtrante (10), et le canal d'entrée d'air (30) est apte à permettre la communication de la cavité filtrante (10) avec la cavité abdominale humaine (F) ; et l'unité de pompe d'entraînement (200) comprend une cavité d'étanchéité (40) et un mécanisme d'entraînement (50), le mécanisme d'entraînement (50) est installé dans la cavité d'étanchéité (40), la cavité d'étanchéité (40) est positionnée dans la cavité filtrante (10), et la cavité filtrante (10) communique avec la cavité d'étanchéité (40), une paroi de la cavité d'étanchéité (40) est pourvue du canal d'évacuation d'air (60), le canal d'évacuation d'air (60) est capable de permettre à la cavité d'étanchéité (40) d'être mise en communication avec la cavité abdominale humaine (F);
la paroi de cavité de la cavité filtrante (10) est munie d'un orifice de communication (131), le canal d'entrée d'air (30) est apte à permettre la communication de l'orifice de communication (131) avec la cavité abdominale humaine (F).
La cavité d'étanchéité (40) comprend un premier corps de couvercle de cavité (41) et un deuxième corps de couvercle de cavité (42), qui forment ensemble une cavité cylindrique, dans laquelle le premier corps de couverture de cavité (41) et le deuxième corps de couverture de cavité (42) ont une forme cylindrique creuse au moins par sections, les sections cylindriques creuses étant reliées les unes aux autres, et le premier corps de couverture de cavité (41) est disposé sous le deuxième corps de couverture de cavité (42), une ouverture supérieure (410) du premier corps de couverture de cavité (41) est en connexion étanche avec une ouverture inférieure (420) du deuxième corps de couverture de cavité (42), et une partie supérieure du deuxième corps de couvercle de cavité (42) est pourvue du canal d'évacuation d'air (60) faisant saillie vers l'extérieur, dans lequel le canal d'évacuation d'air (60) est installé sur le dessus du deuxième corps de couvercle de cavité (42) ; une paroi de cavité du premier corps de couvercle de cavité (41) est pourvue d'un orifice de canal (411), et/ou une paroi de cavité du deuxième corps de couvercle de cavité (42) est pourvue de l'orifice de canal (411), l'orifice de canal (411) est en communication avec l'orifice de communication (131);
l'unité de filtrage (100) comprend un troisième corps de couvercle de cavité (11), un quatrième corps de couvercle de cavité (12), un support interne (13), un support externe (14) et un couvercle d'étanchéité (15), l'élément filtrant (20) est scellé et installé entre le support intérieur (13) et le support extérieur (14), une extrémité de chacun du support interne (13) et du support externe (14) est en connexion étanche avec un fond de couvercle (111) du troisième corps de couvercle de cavité (11), et l'autre extrémité de chacun du support interne (13) et du support externe (14) est en connexion étanche avec le couvercle d'étanchéité (15), le fond de couvercle (111) du troisième corps de couvercle de cavité (11) est en liaison étanche avec le fond de couvercle (111) du premier corps de couvercle de cavité (41), et une ouverture supérieure du troisième corps de couvercle de cavité (11) est en connexion étanche avec une ouverture inférieure du quatrième corps de couvercle de cavité (12) ; le couvercle d'étanchéité (15) est pourvu d'une ouverture (151) communiquant avec le canal d'évacuation d'air (60), et une paroi intérieure de l'ouverture (151) est en connexion étanche avec une paroi extérieure du canal d'évacuation d'air (60 );
le quatrième corps de couvercle de cavité (12) est pourvu d'une première partie de séparation (121) faisant saillie vers l'intérieur, le quatrième corps de couvercle de cavité (12) est pourvu d'un orifice d'évacuation d'air (122) et d'un orifice d'entrée d'air (123) faisant saillie vers l'extérieur ; le couvercle d'étanchéité (15) est pourvu d'une deuxième partie de séparation (152) adaptée et reliée à la première partie de séparation (121), et une cavité formée après que le troisième corps de couvercle de cavité (11) est en connexion étanche avec le quatrième corps de couvercle de cavité (12) est divisée en une cavité d'évacuation d'air (16) et le canal d'entrée d'air (30) par deux parties ; l'orifice d'évacuation d'air (122), la cavité d'évacuation d'air (16) et le canal d'évacuation d'air (60) sont successivement mis en communication ; et l'orifice d'entrée d'air (123) communique avec le canal d'entrée d'air (30).

2. Le dispositif intégré de traitement par circulation de gaz intracavité pour une chirurgie laparoscopique selon la revendication 1, dans laquelle l'unité de pompe d'entraînement (200) comprend en outre une partie de raccordement d'entraînement (70), la pièce de liaison d'entraînement (70) pénètre dans la cavité d'étanchéité (40) et est en liaison étanche avec une paroi de cavité de la cavité d'étanchéité (40), une partie d'extrémité interne de la partie de connexion d'entraînement (70) est connectée au mécanisme d'entraînement (50), et une partie d'extrémité externe de la partie de connexion d'entraînement (70) est connectée de manière amovible à un dispositif d'entraînement externe.

3. Le dispositif de traitement de circulation de gaz intracavitaire intégré pour la chirurgie laparoscopique selon la revendication 2, dans laquelle : la partie de connexion d'entraînement (70) est en connexion étanche avec la paroi de cavité de la cavité d'étanchéité (40) par l'intermédiaire d'un fluide magnétique.
